# EUROPEAN PATENT SPECIFICATION

(11) **EP 0 384 261 B1**
(45) Date of publication and mention of the grant of the patent: **19.05.1993**
(21) Application number: 90102714.4
(22) Date of filing: 12.02.1990
(51) Int. Cl.: C07C 309/80, C07C 303/02

(54) **Process for the preparation of fluorooxy-fluorosulfonyl compounds by direct fluorination of fluoro-beta-sultones**
Verfahren zur Herstellung von Fluor-oxy-fluorsulfonylverbindungen durch direkte Fluorierung von Fluor-beta-sultonen
Procédé pour la préparation de composés fluoro-oxy-fluorosulfonyles par fluorination directe de fluoro-bêta-sultones

(30) Priority: 13.02.1989 IT 1942589
(43) Date of publication of application: 29.08.1990
(73) Proprietor: AUSIMONT S.p.A., I-20121 Milano (IT)
(72) Inventor: Marraccini, Antonio, Dr., I-28040 Dormelletto, Novara (IT); Perego, Gabriele, Dr., I-10015 Ivrea, Torino (IT)
(74) Representative: Barz, Peter, Dr.

(56) References cited:
- GB-A- 1 187 728
- US-A- 3 041 317
- US-A- 4 466 881

## Description

The present invention relates to a process for preparing fluorooxy-fluorosulfonyl compounds according to the following reaction scheme: 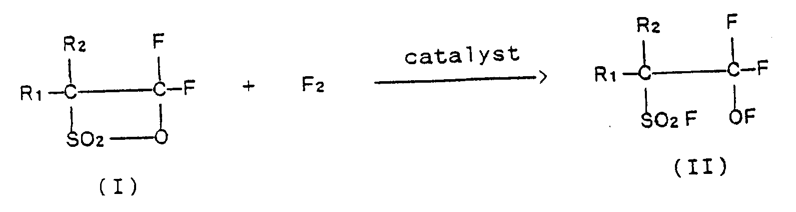
wherein
- R₁ and R₂,: the same or different from each other, represent a fluorine or chlorine atom or an alkyl radical partially or completely halogenated with fluorine and/or chlorine.

The fluorooxy-fluorosulfonyl compounds find several uses. For example, they are used as fluorinating agents. Furthermore, they can be reacted with chlorofluorinated olefins such as, e.g., 1,2-dichlorodifluoro-ethylene, in order to obtain an ether adduct from which by dechlorination a fluorosulfonyl-perhaloalkyl-vinyl-ether can be prepared, which is a monomer for ion-exchange copolymers used as membranes (e.g., for diaphragms for electrolytic cells for sodium hydroxide and chlorine production).

To date there are no processes which make it possible to convert fluoro-β-sultones into the corresponding fluorooxy-fluorosulfonyl compounds in a single step.

It is known that fluoro-β-sultones can be isomerized into the corresponding fluorosulfonyl fluoroacyl fluorides by catalysts, in general of nucleophilic character, such as triethylamine, dibutylether, alkali-metal fluorides, etc., or by heating at 80°C for one hour in an autoclave [Angew. Chemie Int. Ed., 11 (1972) No.7, 583; US-A-4,466,881].

According to the prior art, when one starts from tetrafluoroethane-β-sultone, the isomerization reaction proceeds as follows:

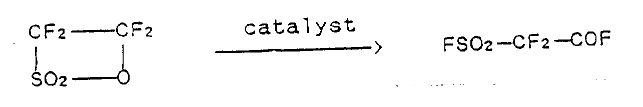

The thus obtained fluorosulfonyl difluoroacetyl fluoride can subsequently be converted into the corresponding fluorooxy compound according to the process of US-A-4,801,409.

An object of the instant invention, therefore, is to provide a process which makes it possible to obtain the above fluorooxy-fluorosulfonyl compounds of formula (II) in a single step by starting from the corresponding fluoro-β-sultones, thus avoiding the step of isomerization into acyl fluorides, i.e., products which per se are sensitive to hydrolysis and which, therefore, require special handling.

Another object is to obtain the above fluorooxy-fluorosulfonyl compounds in high yields and with high purity.

These objects are achieved by means of a process, for preparing fluorooxy-fluorosulfonyl compounds of formula (II): 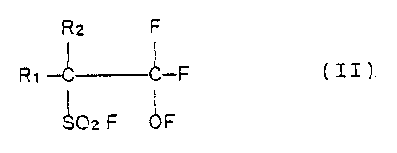
wherein
- R₁ and R₂,: the same or different from each other, represent a fluorine or chlorine atom or an alkyl radical partially or completely halogenated with fluorine and/or chlorine,

by causing a gaseous stream of fluorine and a fluoro-β-sulfone of formula (I): 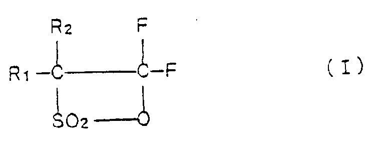
wherein R₁ and R₂ are defined as above, to continuously contact, preferably together with an inert carrier gas, a fluorination catalyst, which catalyst preferably is a fixed-bed catalyst or is suspended in a liquid reaction medium or vehicle inert under the operating conditions. The reaction product is continuously removed in gaseous form from said fluorination catalyst.

The average contact time of the reactants with the catalyst preferably ranges from 1 second to 10 minutes. The temperature is within the range of from -40°C (particularly -30°C) to + 100°C, and the pressure ranges from 50 to 800 kPa.

By operating under the reaction conditions according to the present invention, the fluoro-β-sultones of formula (I) are directly converted, in very high yields, into the fluorooxy-fluorosulfonyl compounds.

The process can be carried out with all of the known types of fluorination catalysts. Preferably, an alkali-metal fluoride and, in particular, cesium fluoride or potassium fluoride, is used.

When a fixed-bed catalyst is employed, said catalyst preferably is supported on a material capable of securing a good heat exchange or is mixed with such a material. Among the materials suitable for the intended purpose, e.g., copper and copper alloys such as brass and Monel can be mentioned.

When the catalyst is suspended in an inert, liquid reaction vehicle or medium the reactants, preferably mixed with an inert diluent gas, are bubbled through said liquid medium which is kept stirred in order to keep the catalyst in homogeneous suspension.

The liquid medium preferably is a perfluoropolyether. Perfluoropolyethers are well-known compounds disclosed, e.g., in GB-A 1,104,482 and in US-A-3,242,218; 3,665,041 and 3,715,378. The use of a catalyst suspended in a liquid medium is sometimes preferred, particularly when a relatively unstable fluorooxy-fluorosulfonyl-perhaloalkane is to be prepared, because the liquid medium secures a better homogeneity of heat exchange, thus preventing local overheating.

Preferably the reaction is carried out at a temperature of from -30°C to +100°C. The operating reaction pressure ranges from 50 to 800 kPa, a pressure slightly higher than atmospheric pressure being preferably used, i.e., a slight overpressure being maintained, which is suitable for causing the gas to flow through the fluorination reactor.

Preferably, the reactants are diluted with a carrier gas which is inert under the reaction conditions. Commonly, the amount of this gas is such that the concentration of the reactants in the gas mixture is within the range of from 5 to 70% by volume.

Preferred examples of the carrier gas are nitrogen, helium, argon and chlorofluorocarbons such as tetrafluoromethane, dichlorotetrafluoroethanes and pentafluorochloroethane.

The molar ratio of fluorine to fluoro-β-sultone preferably ranges from 1.0 to 1.5.

The flow rate of each reactant usually is from 5.10⁻⁶ to 1 mol/hour per gram of catalyst and preferably ranges from 10⁻⁴ to 10⁻¹ mol/hour per gram of catalyst.

In the fluoro-β-sultone used as the starting material, the radicals R₁ and R₂ preferably represent a fluorine atom or a perhaloalkyl radical containing from 1 to 10, particularly 1 to 3 carbon atoms (e.g. CF₃, C₂F₅ and C₃F₇).

The fluoro-β-sultones in which one or both or R₁ and R₂ represent a perfluoroalkyl radical of from 1 to 3 carbon atoms are particularly preferred. When only one of R₁ and R₂ is a C₁₋₃ perfluoroalkyl group, the other one preferably is a fluorine atom.

In the case of tetrafluoroethane-β-sultone (R₁ = R₂ = F), the reaction proceeds according to the following reaction scheme:

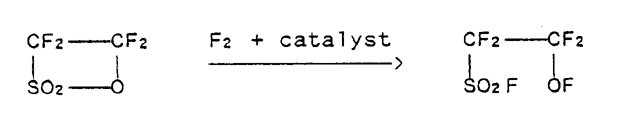

Examples of fluoro-β-sultones suitable for the process according to the present invention are the following:
1) 3,3,4,4-tetrafluoro-2,2-dioxo-1,2-oxathietane;
2) 3,4,4-trifluoro-3-chloro-2,2-dioxo-1,2-oxathietane;
3) 3,4,4-trifluoro-3-trifluoromethyl-2,2-dioxo-1,2-oxathietane;
4) 3,4,4-trifluoro-3-(1,1,2,2,3,3,4,4,5,5,6,6-dodecafluorohexyl)-2,2-dioxo-1,2-oxathietane.

The following non-limitative examples are given in order to illustrate the present invention.

### EXAMPLE 1

250 ml of tetrafluoroethane-β-sultone were introduced into a bubbler of 500 ml capacity and cooled down to 0°C. By means of a candle of sintered glass a stream of 1.0 l/hour of N₂ was passed through said bubbler. The nitrogen stream left the bubbler enriched with vapours of β-sultone. After a further dilution with 10.0 l/hour of N₂, the β-sultone-containing gas stream was mixed with a stream of 1.5 l/hour of F₂, and the mixture was continuously fed to a cylindrical reactor of AISI 316 steel (capacity 130 ml), completely filled with small copper needles mixed with 50 g of(previously ground and thoroughly dried)CsF. The temperature of the reactor was adjusted to the constant temperature of +25°C by circulating a liquid inside the outer jacket of the reactor.

The contact time of the gases with the catalyst was about 70 seconds.

Within 70 hours 44 g of tetrafluoroethane-β-sultone were entrained, as determiend by weighing the bubbler at the beginning and the end of the test run.

The mixture leaving the reactor, analysed by means of an inline I.R. spectrophotometer showed a nearly total disappearance of the typical absorption bands of tetrafluoroethane-β-sultone within the range of from 1900 to 1980 cm⁻¹ and the appearance of the typical absorption bands of fluorooxy-fluorosulfonyl-tetrafluoroethane at 1460 cm⁻¹ (FSO₂-), between 1100 and 1340 cm⁻¹ and at 896 cm⁻¹ (-OF).

The conversion of tetrafluoroethane-β-sultone was higher than 98%.

The ¹⁹F-N.M.R. also was in accordance with the expected structure:δOF = +152.4 ppm.

By gas-mass analysis and ¹⁹F-N.M.R. analysis of the reaction product of fluorooxy-fluorosulfonyl-tetrafluoroethane and 1,2-dichlorodifluoroethylene, it was possible to identify the isolated addition product of formula

FSO₂CF₂CF₂OCFClCF₂Cl,

which further confirmed the chemical nature of the above intermediate fluorooxy compound.

### EXAMPLE 2

To the same reactor as of Example 1, the same mixture of reactant gases was fed under the same experimental conditions, but with a temperature of +80°C maintained inside the fluorination reactor.

Analysis of the reaction products confirmed the conversion of tetrafluoroethane-β-sultone into fluorooxy-fluorosulfonyl-tetrafluoroethane. Once again, a conversion higher than 98% was obtained.

## Claims

1. Process for preparing, in a single step, fluorooxy-fluorosulfonyl compounds of formula (II): 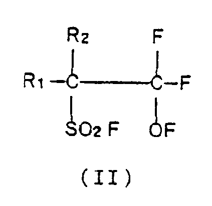 wherein
R₁ and R₂, the same or different from each other, represent a fluorine or chlorine atom or an alkyl radical partially or completely halogenated with fluorine and/or chlorine,
characterized in that a gaseous stream of a mixture of fluorine and a fluoro-β-sultone of formula (I): 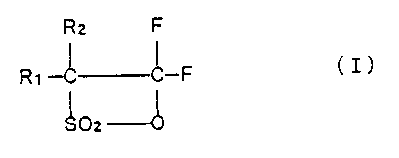 wherein R₁ and R₂ are as defined above, is caused to continuously contact a fluorination catalyst at temperatures of from -40°C to +100°C and at pressures of from 50 to 800 kPa, the reaction product being continuously removed from said fluorination catalyst.

2. Process according to claim 1, wherein the fluorination catalyst is an alkali-metal fluoride, particularly potassium fluoride and/or cesium fluoride.

3. Process according to any one of the preceding claims, wherein the fluorination catalyst is a fixed-bed catalyst, particularly a fixed-bed catalyst supported on or mixed with a material capable of providing good heat exchange.

4. Process according to claim 3 wherein the material capable of providing good heat exchange is copper or a copper alloy.

5. Process according to any one of claims 1 and 2, wherein the fluorination catalyst is suspended in a liquid reaction medium inert under the reaction conditions, particularly a perfluoropolyether.

6. Process according to one or more of the preceding claims, wherein the gaseous stream of the reactants is diluted with a carrier gas which is inert under the reaction conditions.

7. Process according to claim 7, wherein the inert carrier gas is selected from nitrogen, helium, argon, fluorocarbons and fluorochlorocarbons.

8. Process according to any one of claims 6 and 7, wherein the inert carrier gas is used in an amount of from 30 to 95% by volume, based on the volume of the gas mixture.

9. Process according to one or more of the preceding claims, wherein the molar ratio of fluorine to fluoro-β-sultone ranges from 1:1 to 1.5:1.

## Patentansprüche

1. Verfahren zur einstufigen Herstellung von Fluoroxyfluorsulfonylverbindungen der Formel (II) 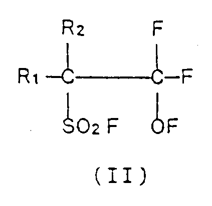 worin R₁ und R₂ gleich oder unterschiedlich ein Fluor- oder Chloratom oder einen Alkylrest, der ganz oder teilweise mit Fluor und/oder Chlor halogeniert ist, bedeuten,
dadurch gekennzeichnet, daß ein gasförmiger Strom einer Mischung aus Fluor und einem Fluor-β-sulton der Formel (I) 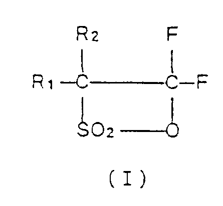 worin R₁ und R₂ wie oben definiert sind, kontinuierlich mit einem Fluorierungskatalysator bei Temperaturen von -40°C bis +100°C und Drücken von 50 bis 800 kPa in Kontakt gebracht wird, wobei man das Reaktionsprodukt kontinuierlich von dem Fluorierungskatalysator entfernt.

2. Verfahren nach Anspruch 1, worin der Fluorierungskatalysator ein Alkalimetallfluorid, insbesondere Kaliumfluorid und/oder Cäsiumfluorid, ist.

3. Verfahren nach einem der vorangehenden Ansprüche, worin der Fluorierungskatalysator ein Festbettkatalysator ist, insbesondere ein Festbettkatalysator, der mit einem Material geträgert oder vermischt ist, welches einen guten Wärmeaustausch ermöglicht.

4. Verfahren nach Anspruch 3, worin das Material, welches einen guten Wärmeaustausch ermöglicht, Kupfer oder eine Kupferlegierung ist.

5. Verfahren nach einem der Ansprüche 1 oder 2, worin der Fluorierungskatalysator in einem flüssigen Reaktionsmedium suspendiert ist, das unter den Reaktionsbedingungen inert ist, insbesondere einem Perfluorpolyether.

6. Verfahren nach einem oder mehreren der vorangehenden Ansprüche, worin der gasförmige Strom der Reaktanten mit einem Trägergas, das unter den Reaktionsbedingungen inert ist, verdünnt wird.

7. Verfahren nach Anspruch 7, worin das inerte Trägergas ausgewählt ist aus Stickstoff, Helium, Argon, Fluorkohlenstoffen und Fluorchlorkohlenstoffen.

8. Verfahren nach einem der Ansprüche 6 und 7, worin das inerte Trägergas in einer Menge von 30 bis 95 Volumenprozent, bezogen auf das Volumen des Gasgemisches, verwendet wird.

9. Verfahren nach einem oder mehreren der vorangehenden Ansprüche, worin das Molverhältnis von Fluor zu Fluor- β-sulton 1:1 bis 1,5:1 beträgt.

## Revendications

1. Procédé pour préparer en une seule étape les composés fluoro-oxy-fluorosulfonyle de formule (II) : 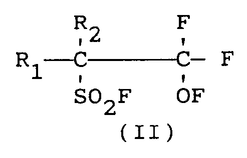 dans lequel :
R₁ et R₂ identiques ou différents les uns des autres, représentent un atome de fluor ou de chlore ou un radical alkyle partiellement ou complètement halogéné par du fluor et/ou du chlore.
ce procédé étant caractérisé en ce qu'un courant gazeux d'un mélange de fluor et de fluoro-β-sultone de formule (I) : 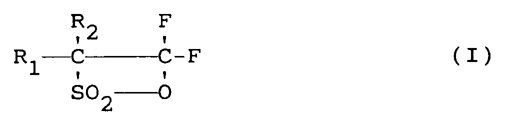 dans laquelle
R₁ et R₂, sont tels que définis ci-dessus,
et est amené à entrer en contact continu avec un catalyseur de fluoration à une température comprise entre -40°C et + 100°C et sous une pression comprise entre 50 et 800 kPa, le produit de réaction étant continuellement séparé du catalyseur de fluoration précité.

2. Procédé selon la revendication 1, caractérisé en ce que le catalyseur de fluoration est un fluorure de métal alcalin, notamment le fluorure de potassium et/ou le fluorure de césium.

3. Procédé selon l'une quelconque des revendications précédentes, caractérisé en ce que le catalyseur de fluoration est un catalyseur en lit fixe, notamment un catalyseur en lit fixe supporté ou mélangé avec une matière susceptible de permettre un bon échange thermique.

4. Procédé selon la revendication 3, caractérisé en ce que la matière susceptible d'assurer un bon échange thermique est du cuivre ou un alliage de cuivre.

5. Procédé selon l'une quelconque des revendications 1 et 2, caractérisé en ce que le catalyseur de fluoration est mis en suspension dans un milieu de réaction liquide inerte dans les conditions de réaction, notamment un perfluoropolyéther.

6. Procédé selon l'une quelconque des revendications précédentes, dans lequel le courant gazeux des réactifs est dilué dans un gaz de support qui est inerte dans les conditions réactionnelles.

7. Procédé selon la revendication 6, dans lequel le gaz support inerte est choisi dans le groupe comprenant l'azote, l'hélium, l'argon, les dérivés fluorocarbonés et les dérivés fluorochlorocarbonés.

8. Procédé selon l'une des revendications 6 et 7, dans lequel le gaz support inerte est utilisé en une quantité comprise entre 30 et 95% en volume, exprimé par rapport au volume du mélange de gaz.

9. Procédé selon l'une quelconque des revendications précédentes, dans lequel le rapport molaire fluor/fluoro-β-sultone est compris entre 1/1 à 1,5/1.
